# DEMANDE DE BREVET EUROPEEN

(11) **EP 2 606 921 A1**
(43) Date de publication de la demande: **26.06.2013**
(21) Numéro de dépôt: 11010040.1
(22) Date de dépôt: 21.12.2011
(51) Int. Cl.: A61M 1/34, A61M 1/36

(54) **Dispositif d'épuration du sang par circulation extracorporelle**

(71) Demandeur: Infomed SA, 1227 Acacias (CH)
(72) Inventeur: Favre, Olivier, 1226 Thônex (CH)
(74) Mandataire: Micheli & Cie SA

(57) **Abrégé**

Dispositif d'épuration du sang comportant une boucle de circulation extracorporelle du sang composé d'un élément (1) qui extrait le plasma du sang et des moyens de circulation du plasma ainsi extrait dans des moyens d'épuration du plasma (3,4,5,6 ou 8). Le dispositif comporte de plus une colonne (2) dans ladite boucle de circulation du sang.

## Description

La présente invention se rapporte à un dispositif d'épuration du sang de patients présentant une pathologie qui nécessite de réduire les quantités circulantes de plusieurs substances possédant des caractéristiques physico-chimiques différentes. La maladie de Crohn, l'arthrite rhumatoïde, certaines formes de psoriasis ou encore la maladie de Behçet appartiennent, entre autres, à cette catégorie du fait que les systèmes immunitaire et inflammatoire interagissent pour créer des inflammations chroniques qui progressent jusqu'à fortement réduire la qualité de vie des patients.

Les mécanismes d'interaction entre les leucocytes et les anticorps d'une part, pour le système immunitaire, et les cytokines d'autre part, pour le système inflammatoire, ont été étudiés et font l'objet de plusieurs publications qui éclairent certains aspects de ces interactions tout en mettant en évidence leur incroyable complexité. La somme de connaissances de l'ensemble des études concernées ne décrit au final que de manière simplifiée les mécanismes existants. L'importance de chaque substance dans l'inflammation ainsi que leur stimulation mutuelle pour se multiplier de manière pathologique a cependant été mise en évidence ; ainsi parmi les différents leucocytes existants, ce sont principalement les monocytes et les granulocytes qui sont impliqués dans les pathologies mentionnées ci-dessus, les lymphocytes jouant un rôle mineur.

Dans les cas les plus graves des pathologies décrites ci-dessus, une épuration du sang des patients par circulation extracorporelle est réalisée. Dans les modes classiques de traitement, deux approches existent qui visent principalement à réduire la réaction immunitaire en épurant des leucocytes ou des anticorps. L'épuration de cytokines n'a pas été pratiquée dans ces cas.

Pour retirer des leucocytes du sang, il y a deux techniques, l'une procédant par filtration et l'autre par adsorption. Ces méthodes ont été développées dans les années 90. Dans les deux cas, on place l'élément d'épuration sur le circuit de circulation extracorporelle de sang, lequel traverse ledit élément et en sort avec une concentration réduite de leucocytes par rapport à la valeur d'entrée dans l'élément. Cet élément qui possède uniquement une entrée et une sortie pour le sang et retient certaines substances spécifiques est dénommé « colonne ». Dans le cas d'une colonne agissant par filtration, on utilise le fait que les leucocytes sont les plus grosses substances contenues dans le sang pour les retenir mécaniquement, alors que les globules rouges et autres protéines passent à travers la colonne. Un exemple de tel élément est le Cellsorba de la société Asahi Kasei qui a été techniquement décrit dans le brevet US4476023 mais pour une application différente de celle présentée ici. Dans le cas de l'élément agissant par adsorption on utilise l'affinité des leucocytes pour la matière contenue dans l'élément, matière sur laquelle les leucocytes « adhèrent » alors que les autres éléments restent libres de circuler dans le sang. Un exemple de tel élément est l'Adacolumn de la société Jimro objet du brevet US6498007.

Ces deux méthodes d'épuration présentent des résultats cliniques analogues malgré des taux de retenue des leucocytes qui sont différents. A noter que des techniques par centrifugation sont aussi parfois utilisées mais avec des résultats cliniques inférieurs.

Pour retirer des anticorps du sang il existe trois techniques. Premièrement l'échange plasmatique, dans lequel le plasma extrait du sang est jeté alors qu'un fluide de compensation contenant de l'albumine est injecté pour compenser les pertes, deuxièmement la filtration en cascade au cours de laquelle le plasma extrait du sang passe à travers un filtre qui retient les anticorps avant d'être retourné au sang et troisièmement la plasma adsorption qui utilise sur le circuit de plasma une colonne adsorbante conçue pour retenir les anticorps.

Ces trois techniques ont été inventées dans les années 70. L'échange plasmatique est pratiqué aujourd'hui à raison de plusieurs centaines de milliers de traitements par année dans le monde. Plusieurs dispositifs existent pour réaliser l'échange plasmatique, ils séparent le plasma du sang par filtration ou par centrifugation alors que l'équilibre des volumes entre le plasma jeté et la solution de compensation est réalisé en utilisant soit des balances soit le nombre de tour des pompes respectives. La filtration en cascade est utilisée aujourd'hui à raison de plusieurs dizaines de milliers de traitements par an. Plusieurs appareils existent qui permettent de réaliser cette technique. La plasma adsorption est pratiquée à raison de quelques milliers de traitements par an, avec les différents modèles de colonne adsorbantes disponibles. A nouveau, ces trois méthodes présentent des résultats cliniques analogues et globalement légèrement inférieurs à celles consistant à retirer des leucocytes.

Les méthodes d'épuration du sang par circulation extracorporelle décrites ci-dessus restent inefficaces pour les patients les plus gravement atteints ainsi que pour 20 à 50% des patients atteints à des degrés moyens. Pour pallier à cet inconvénient et pouvoir soigner tous les patients un dispositif d'épuration du sang par circulation extracorporelle plus élaboré, objet de la présente invention, doit être réalisé. La raison des limites des dispositifs existants est qu'ils ne permettent de retirer que l'un des éléments à la fois, soit les leucocytes, les anticorps ou les cytokines; il en résulte que les autres éléments, qui restent en surnombre, activent ensuite la régénération de ce qui a été retiré. Il serait donc utile de pouvoir retirer plusieurs catégories d'éléments en un seul passage de sang afin de prévenir cette cascade de réactions qui empêche de guérir, et même de réduire, la pathologie.

Le fait qu'en pratique les systèmes disponibles ne retirent qu'une seule catégorie de substance à la fois provient d'un cumul de différences dans leurs caractéristiques physico-chimiques et donc dans les moyens possibles pour les retirer du sang. Les leucocytes, de par leur grande taille, ne sont pas présents dans le plasma et doivent donc être retirés directement du sang. Les anticorps peuvent en théorie être adsorbés directement dans le sang mais on préfère les retirer du plasma pour éviter des interactions entre matériel adsorbant et sang. On les retire ainsi du sang soit en jetant le plasma (échange plasmatique), soit par filtration en cascade soit par adsorption. Les cytokines sont préférablement retirées du plasma par adsorption car le volume à traiter pour être efficace est beaucoup plus important que ce que l'échange plasmatique ne permet. Finalement, même si à priori toutes ces substances peuvent être retirées par adsorption, les matériaux utilisables et leurs méthodes de production sont différents.

Le but de la présente invention est de remédier, au moins en partie, aux inconvénients susmentionnés.

Pour ce faire, la présente invention vise un dispositif permettant de retirer du sang des patients non plus seulement une catégorie de substance impliquée dans la cascade de réactions menant à la pathologie, comme c'est le cas dans les techniques utilisées jusqu'à ce jour, mais simultanément au moins deux catégories différentes comme par exemple les leucocytes et les anticorps ou les leucocytes et les cytokines ou les anticorps et les cytokines voire, les leucocytes, les anticorps et les cytokines.

Il existe certains dispositifs qui retirent des substances différentes de par leur nature mais sont impliqués dans une même pathologie. On peut citer le brevet EP2295092 de Bellco qui comporte sur la boucle de circulation extracorporelle de sang un hémofiltre et un hémodialyseur, le premier permettant de retirer de l'eau et des toxines de poids moléculaire élevé pendant que le second fait de même avec les petites molécules. Un autre exemple est le brevet US4683889 qui décrit un dispositif comprenant sur la boucle de sang un dispositif d'irradiation UV suivi d'une centrifugeuse utilisée pour retirer des lymphocytes, l'ensemble étant destiné à lutter contre la leucémie. Un autre exemple est le brevet US6193681 qui comporte sur la boucle de sang un dispositif d'irradiation par UV suivi d'un hémofiltre pour le traitement du sepsis. Le brevet US20040143207 décrit quant à lui une boucle de sang comprenant un hémofiltre et un oxygénateur, cette invention permettant de traiter des patients atteints de défaillance rénale et respiratoire.

On voit ainsi que divers dispositifs permettent déjà, par l'utilisation de plusieurs moyens d'actions situés en série sur la boucle de circulation extracorporelle de sang, de retirer des substances présentant des caractéristiques physico-chimiques différentes.

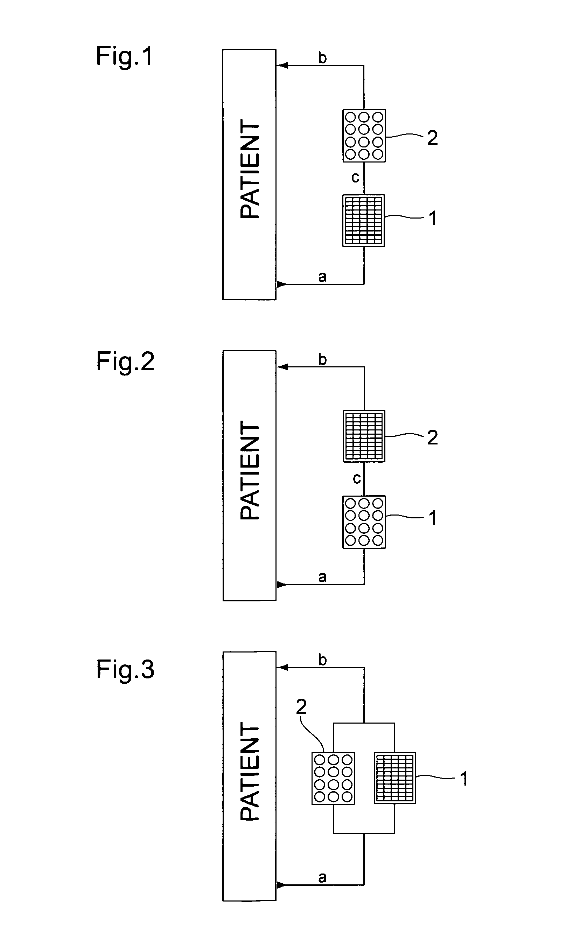

Un appareil selon la présente invention possède, comme représenté sur les figures 1 à 8 une boucle de circulation extracorporelle du sang sur laquelle sont placés un élément permettant d'extraire le plasma du sang et une colonne servant par exemple à retenir des leucocytes. Un circuit de circulation du plasma comprenant des moyens permettant d'éliminer d'autres substances, par exemple des anticorps, des cytokines ou les deux, complète le dispositif.

Un avantage essentiel de cette invention réside dans le fait qu'elle permet, par rapport aux techniques existantes, de réduire significativement la quantité et la diversité des substances intervenant dans les pathologies décrites ci-dessus, permettant ainsi une plus grande efficacité clinique.

Les variantes de réalisation selon l'invention sont montrées sur les figures 1 à 8. Elles possèdent toutes une boucle de circulation extracorporelle de sang composée d'un conduit d'extraction du sang du patient (a) sur lequel se trouve un élément de séparation sang-plasma (1) relié par un conduit (c) à une colonne (2), le sang étant ensuite retourné au patient par un conduit (b). Un circuit de ce type est bien sûr complété par des moyens de mesures des pressions, une ou plusieurs pompes, un détecteur d'air, tous éléments qui sont décrits dans de nombreuses publications et normes techniques qui s'appliquent à la présente dans toutes les variantes connues sans en affecter sa principale caractéristique.

Différentes configurations de la boucle de circulation du sang sont possibles et toutes conviennent à réaliser un appareil selon la présente invention. A titre d'exemple on peut citer un dispositif comprenant : une aiguille d'extraction du sang, un tube équipé d'un connecteur pour la lecture de la pression d'extraction reliant ladite aiguille à une pompe péristaltique qui force le sang à travers la boucle de sang, un filtre à fibres creuses possédant une porosité telle que du plasma puisse être extrait du sang, un tube de retour de sang connecté à une aiguille de retour du sang et comprenant un piège à bulles et un connecteur pour la lecture de la pression de retour. Le tube de retour passe dans un détecteur de bulles d'air que suit un clamp, l'ensemble permettant de prévenir une éventuelle injection d'air au patient. Des variantes d'un tel dispositif peuvent comprendre un cathéter à double lumen en remplacement des aiguilles et/ou un séparateur sang/plasma agissant par centrifugation en remplacement du filtre. De tels dispositifs existent depuis les années 70-80. A l'une ou l'autre version de cette boucle de circulation connue, une boucle de circulation du sang selon l'invention comprendra non pas un mais deux éléments d'épuration, un filtre de plasma 1 et une colonne 2 retenant des substances spécifiques, comme par exemple des leucocytes.

Les éléments 1 et 2 peuvent être placés de manière indifférente sur la boucle de sang sans que cela n'influence le fonctionnement de la présente invention. L'élément 2 peut par exemple être positionné en aval de l'élément 1 (figure 1), en amont de l'élément 1 (figure 2) ou les deux éléments peuvent être placés en parallèle (figure 3), variantes qui font toutes partie de la présente invention.

A cette boucle de circulation du sang comportant une colonne 2 qui retient des éléments spécifiques et un élément 1 qui extrait le plasma, on ajoute un ou plusieurs moyens pour retirer des substances du plasma extrait.

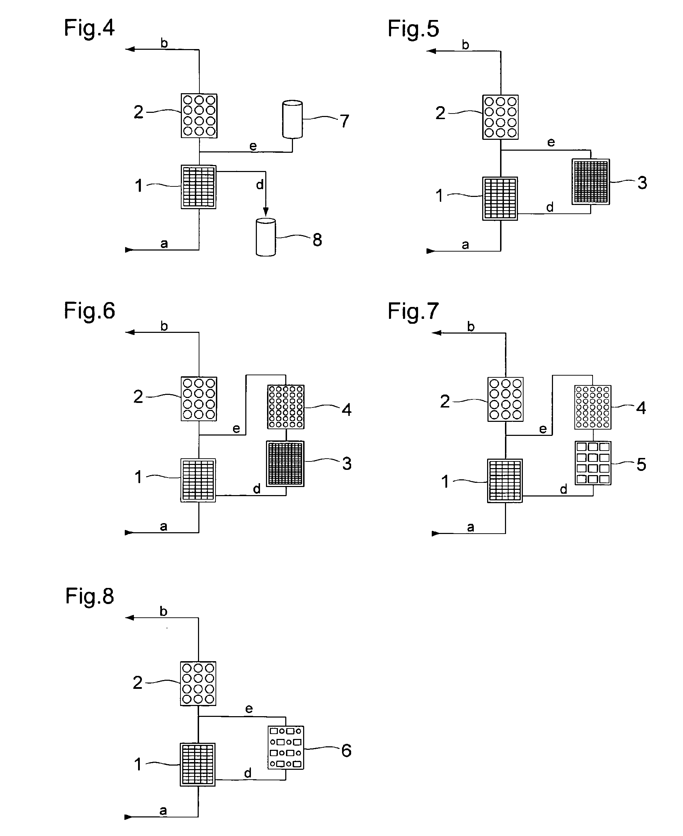

La boucle de circulation du plasma d'un appareil fonctionnant conformément à la présente invention, représentée sur l'une des figures 4 à 8, est composée d'un tube (d) permettant de faire circuler le plasma depuis le séparateur sang-plasma 1, de moyens de circulation connus et non représentés du plasma dans la boucle de plasma, d'un ou plusieurs éléments (3,4,5,6,8) destinés à retirer diverses substances du plasma et d'un tube (e) de retour du plasma ainsi épuré dans la boucle de circulation du sang du patient.
La figure 4 montre un dispositif dans lequel le plasma est jeté dans un réservoir (8) alors qu'un fluide de compensation contenu dans un réservoir (7) est ajouté dans le circuit de circulation du sang.
La figure 5 montre un système dans lequel le plasma passe par un filtre (3) pour en retirer des anticorps.
La figure 6 montre un dispositif comprenant un filtre (3) destiné à retenir des anticorps suivi d'un élément (4) d'adsorption de cytokines.
La figure 7 présente une version du dispositif dans laquelle 2 colonnes d'adsorption (4,5) sont placées sur la ligne de plasma, l'une retenant des anticorps et l'autre des cytokines.
La figure 8 comporte une cartouche unique (6) qui est conçue de manière à retenir à la fois des anticorps et des cytokines.

Ces boucles de plasma illustrées aux figures 4 à 8 dans une configuration de la boucle de circulation extracorporelle selon la figure 1, où l'extraction et le retour de plasma se font en amont de la colonne 2, peuvent bien entendu être aussi appliquées aux boucles de circulation extracorporelle de sang selon les figures 2 et 3. De même le retour de plasma dans la boucle de circulation du sang peut se faire sur un segment quelconque (a), (b) ou (c).

## Revendications

1. Dispositif d'épuration du sang comportant une boucle de circulation extracorporelle du sang composé d'un élément (1) qui extrait le plasma du sang et des moyens de circulation du plasma ainsi extrait dans des moyens d'épuration du plasma (3,4,5,6 ou 8) **caractérisé en ce qu'**il comporte de plus une colonne (2) dans ladite boucle de circulation du sang.

2. Dispositif selon la revendication 1 **caractérisé en ce que** la colonne (2) retient des leucocytes.

3. Dispositif selon la revendication 1 **caractérisé en ce que** la colonne (2) et l'élément (1) sont placés en série sur la boucle de circulation du sang.

4. Dispositif selon la revendication 1 **caractérisé en ce que** la colonne (2) et l'élément (1) sont placés en parallèle sur la boucle de circulation du sang.

5. Dispositif selon la revendication 1 **caractérisé en ce que** le plasma est jeté dans un réservoir (8) puis compensé en tout ou partie par un liquide de substitution contenu dans un réservoir (7).

6. Dispositif selon la revendication 1 **caractérisé en ce que** le plasma extrait est circulé à travers un filtre (3) puis retourné dans la boucle de circulation du sang.

7. Dispositif selon la revendication 1 **caractérisé en ce que** le plasma extrait est circulé à travers un filtre (3) et une cartouche adsorbante (4) puis retourné dans la boucle de circulation du sang.

8. Dispositif selon la revendication 1 **caractérisé en ce que** le plasma extrait est circulé à travers deux cartouches adsorbantes (4,5) puis retourné dans la boucle de circulation du sang.

9. Dispositif selon la revendication 1 **caractérisé en ce que** le plasma extrait est circulé à travers une seule cartouche adsorbante (6) retenant des anticorps et des cytokines puis retourné dans la boucle de circulation du sang.
